(19) Europäisches Patentamt
European Patent Office
Office européen des brevets

(11) EP 4 247 399 B1

(12) EUROPEAN PATENT SPECIFICATION

(45) Date of publication and mention of the grant of the patent:
**16.09.2026 Bulletin 2026/38**

(21) Application number: **21847528.3**

(22) Date of filing: **22.11.2021**

(51) International Patent Classification (IPC):
***A61K 36/752*** (2006.01) ***A23L 2/06*** (2006.01)
***A61P 3/06*** (2006.01) ***A61P 3/10*** (2006.01)
***A61P 9/10*** (2006.01) ***A61P 1/16*** (2006.01)

(52) Cooperative Patent Classification (CPC):
**A61P 9/10; A23L 2/06; A61K 36/752; A61P 1/16; A61P 3/06; A61P 3/10**

(86) International application number:
**PCT/IB2021/060817**

(87) International publication number:
**WO 2022/107093 (27.05.2022 Gazette 2022/21)**

# (54) COMPOSITION COMPRISING NATURAL EXTRACTS AND USES THEREOF

ZUSAMMENSETZUNG MIT NATÜRLICHEN EXTRAKTEN UND VERWENDUNGEN DAVON

COMPOSITION COMPRENANT DES EXTRAITS NATURELS ET SES UTILISATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.11.2020 IT 202000027963**

(43) Date of publication of application:
**27.09.2023 Bulletin 2023/39**

(73) Proprietor: **Esserre Pharma Srl**
**00191 Roma (IT)**

(72) Inventors:
- **RICCIONI, Costanza Valentina**
  **00191 Roma (RM) (IT)**
- **SQUILLACE GRECO, Amedeo**
  **00191 Roma (RM) (IT)**
- **DE FILIPPIS, Daniele**
  **00191 Roma (RM) (IT)**

(74) Representative: **Pace Napoleone, Maria et al**
**De Simone & Partners S.r.l.**
**Via Giulio Caccini, 1**
**00198 Roma (IT)**

(56) References cited:
**EP-A1- 2 719 287**
**WO-A1-2010/041290**
**WO-A1-2015/136441**
**US-A- 5 877 208**
**US-A1- 2011 223 271**

- **MUSOLINO VINCENZO ET AL: "Bergamot Polyphenols Improve Dyslipidemia and Pathophysiological Features in a Mouse Model of Non-Alcoholic Fatty Liver Disease.", vol. 10, no. 1, 13 February 2020 (2020-02-13), pages 2565, XP055823628, Retrieved from the Internet <URL:https://www.nature.com/articles/s41598-020-59485-3.pdf> DOI: 10.1038/s41598-020-59485-3**
- **BALLISTRERI GABRIELE ET AL: "Evaluation of lipid and cholesterol-lowering effect of bioflavonoids from bergamot extract", NATURAL PRODUCT RESEARCH, 1 May 2020 (2020-05-01), GB, pages 1 - 6, XP055823763, ISSN: 1478-6419, DOI: 10.1080/14786419.2020.1768085**
- **DATABASE WPI Week 201240, Derwent World Patents Index; AN 2012-B02144, XP002803651**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 247 399 B1

- LEONARDO DI DONNA ET AL: "Hypocholesterolaemic activity of 3-hydroxy-3-methyl-glutaryl flavanones enriched fraction from bergamot fruit (Citrus bergamia): "In vivo" studies", JOURNAL OF FUNCTIONAL FOODS, vol. 7, 1 February 2014 (2014-02-01), NL, pages 558 - 568, XP055194428, ISSN: 1756-4646, DOI: 10.1016/j.jff.2013.12.029
- VINCENZO MOLLACE ET AL: "Hypolipemic and hypoglycaemic activity of bergamot polyphenols: From animal models to human studies", FITOTERAPIA, vol. 82, no. 3, 4 November 2010 (2010-11-04), pages 309 - 316, XP028365047, ISSN: 0367-326X, [retrieved on 20101104], DOI: 10.1016/J.FITOTE.2010.10.014
- GLIOZZI MICAELA ET AL: "Bergamot polyphenolic fraction enhances rosuvastatin-induced effect on LDL-cholesterol, LOX-1 expression and protein kinase B phosphorylation in patients with hyperlipidemia", INTERNATIONAL JOURNAL OF CARDIOLOGY, ELSEVIER, AMSTERDAM, NL, vol. 170, no. 2, 8 September 2013 (2013-09-08), pages 140 - 145, XP028788518, ISSN: 0167-5273, DOI: 10.1016/J.IJCARD.2013.08.125
- MONICA LEOPOLDINI ET AL: "On the Inhibitor Effects of Bergamot Juice Flavonoids Binding to the 3-Hydroxy-3-methylglutaryl-CoA Reductase (HMGR) Enzyme", JOURNAL OF AGRICULTURAL AND FOOD CHEMISTRY, vol. 58, no. 19, 13 October 2010 (2010-10-13), pages 10768 - 10773, XP055194516, ISSN: 0021-8561, DOI: 10.1021/jf102576j
- ELZBIETA JANDA ET AL: "Molecular mechanisms of lipid- and glucose-lowering activities of bergamot flavonoids", PHARMANUTRITION, vol. 4, 1 October 2016 (2016-10-01), pages S8 - S18, XP055512854, ISSN: 2213-4344, DOI: 10.1016/j.phanu.2016.05.001
- ERIN E. MULVIHILL ET AL: "Citrus Flavonoids as Regulators of Lipoprotein Metabolism and Atherosclerosis", ANNUAL REVIEW OF NUTRITION., vol. 36, no. 1, 17 July 2016 (2016-07-17), US, pages 275 - 299, XP055475758, ISSN: 0199-9885, DOI: 10.1146/annurev-nutr-071715-050718
- GIGLIO ROSARIA VINCENZA ET AL: "The effect of bergamot on dyslipidemia", PHYTOMEDICINE, ELSEVIER, AMSTERDAM, NL, vol. 23, no. 11, 30 December 2015 (2015-12-30), pages 1175 - 1181, XP029687153, ISSN: 0944-7113, DOI: 10.1016/J.PHYMED.2015.12.005
- MINI ET AL: "Bergamot (Citrus bergamia Risso) Flavonoids and Their Potential Benefits in Human Hyperlipidemia and Atherosclerosis: An Overview", REVIEWS IN MEDICINAL CHEMISTRY, 1 January 2016 (2016-01-01), pages 619 - 629, XP055786110, Retrieved from the Internet <URL:https://www.eurekaselect.com/132938/article> [retrieved on 20210316]

## Description

### FIELD OF THE INVENTION

The present invention relates to a composition consisting of one or more plant extracts and comprising a flavanones content of at least 40% by weight with respect to the total weight of the composition, preferably said composition consists of two or three extracts mixed or combined and/or preferably wherein the flavanones content does not exceed 60% by weight with respect to the total weight of the composition, said composition comprising: a) 35-40% w/w of non-HMG flavanones (3-hydroxy-3-methyl-glutaryl) and b) at least 5% w/w of HMG flavanones, preferably wherein said composition is in micronized form

The invention also relates to the use of such a composition for preventing and/or treating cardiometabolic diseases, dyslipidaemias, insulin resistance, type II diabetes, NAFLD/NASH, hypertension, obesity, visceral fat, for counteracting disorders/risk factors typical of obesity and cardiovascular diseases, such as glycemia, triglycerides, cholesterol.

### BACKGROUND ART

Preparations of bergamot extracts titrated in total flavanones exist on the market, used in the food field in the form of dietary supplements. However, said extracts are characterized by a low titration in flavanones and/or by a wide variability of the titration among batches, due to both the starting raw material (for example different concentration of flavanones in the fruit, variability due to balsamic time, etc.) and to the production methods and conditions of the extracts (extraction on resins/membranes/spray drying). Therefore, with the presently known methods there are difficulties in obtaining a standardization of the obtained preparations.

In particular, the variability among the extract batches largely relates to the HMG flavanones, i.e., the hydroxy-methyl-glutaryl derivatives of the starting flavanones (naringin, naringenin).

To demonstrate the variability among batches as such in bergamot preparations, the following table shows the differences in the non-HMG flavanones and HMG flavanones content in the different batches of a bergamot "pastazzo" preparation obtained from the same manufacturer.

Table 1

| | Batch 1/19 | Batch 2/19 | Batch 3/19 |
|---|---|---|---|
| Non-HMG Flavanones (% w/w) | 20.9 | 28.1 | 25.2 |
| HMG flavanones (% w/w) | 3.7 | 4.5 | 4.3 |

Since in nutraceutical preparations containing "botanicals" the effectiveness of the final product in tablets/capsules, etc. (which contains a defined dose of extract, for example 200 mg) depends on the concentration of the active ingredients in the extract, and since in the case of bergamot it is plausible that it is due to the concentration in HMG flavanones (Di Donna et al., J Nat Prod. 2009; Leopoldini et al.,J. Agric. Food Chem. 2010) for the cholesterol-lowering effect and to the non-HMG flavanones (Janda et al. PharmaNutrition, 2016; Pu et al., Arch Biochem Biophys., 2012) for the insulin-sensitizing effect, if there is variability among the raw material batches in HMG and non-HMG flavanones concentrations, the finished product will be more or less effective. Therefore, there is still a need for a method capable of allowing the production of extracts which have minimal or no variability of the titration between batches and of the extracts thus obtained.

### SUMMARY OF THE INVENTION

The present inventors have surprisingly found a preparation in solid, semi-solid or liquid form of plant origin characterized by a standardized concentration of non-HMG flavanones (3-Hydroxy-3-methyl-glutaryl) (Naringin, Neoeriocitrin, Neoesperidin) and HMG (Brutieridin, Melitidin) in the w/w amounts of 35-40%, preferably 38% (Non-HMG flavanones) and at least 7% (HMG flavanones) for a total of at least 45% flavanones.

The present inventors have also surprisingly found a preparation in solid, semi-solid or liquid form of plant origin characterized by a standardized concentration of non-HMG flavanones (3-Hydroxy-3-methyl-glutaryl) (Naringin, Neoeriocitrin, Neoesperidin) and HMG (Brutieridin, Melitidin) in the w/w amounts of 35-40%, preferably 35% (Non-HMG flavanones) and at least 5% (HMG flavanones) for a total of at least 40% flavanones, where said preparation is preferably in micronized form.

The preparation according to the invention can be obtained by several possible methods, for example:

- mixing bergamot extracts obtained from different matrices: juice and/or "pastazzo" (residues of skins, pulp and seeds after pressing the fruit and eliminating epicarp) and/or albedo from different extraction methods known to those skilled in the art (e.g., resins and membranes, KOH, supercritical $EtOH/H_2O/CO_2$);
- mixing different extracts of different citrus fruits (e.g., bergamot, grapefruit, chinotto, bitter orange or pomelo or combinations thereof) each containing different flavanones and mixed in different proportions.
The preparation according to the present invention can be used in the treatment of cardiometabolic diseases, dyslipidaemias, insulin resistance, type II diabetes, NAFLD (non-alcoholic hepatic steatosis)/NASH (non-alcoholic steatohepatitis) and/or hypertension.

Cardiovascular diseases (diabetes mellitus, stroke, heart attack, atherosclerosis, etc.) are the leading cause of morbidity throughout the world, particularly in Western and more "developed" nations, but with an increasing number of cases also in developing countries. The correlation between plasma LDL cholesterol levels and cardiovascular risk has been extensively demonstrated in both observational and intervention studies.

The pharmacological therapy of choice for dyslipidaemias is represented by statins, inhibitors of the enzyme hydroxymethylglutaryl- coenzyme A- reductase (HMG-CoA-reductase). These act by inhibiting the endogenous biosynthesis of cholesterol in the liver, blocking the conversion of 3-hydroxy-3-methylglutaryl-CoA (HMG-CoA) into mevalonic acid, a cholesterol precursor. Commercially available molecules are simvastatin, lovastatin, pravastatin, atorvastatin, fluvastatin, and rosuvastatin.

Statins can be considered safe medicines; the main side effects are changes in liver function, effects on the muscles (myalgia and myopathy) and, more rarely, rhabdomyolysis.

The most common manifestations of side effects such as myalgia and myopathy and the condition of statin intolerance are considered the main causes of therapy discontinuation.

Other drugs used in the treatment of dyslipidaemias include fibrates, ion exchange resins, ezetimibe.

Type II diabetes mellitus is a clinical condition characterized by a combination of impaired insulin secretion and peripheral insulin resistance. The consequence of a lack of compensation with increased insulin production by pancreatic beta cells is an increase in blood glucose which can lead to the development of complications such as retinopathy, nephropathy, neuropathy and atherosclerosis. Around 415 million people worldwide suffer from diabetes mellitus, a number which is expected to increase to 650 million in 2040.

The drug of choice in the treatment of diabetes is metformin (biguanide class) which improves the sensitivity of tissues to insulin. The mechanism of action of metformin seems to comprise the activation of AMPK (adenosine monophosphate-activated protein kinase) which plays a fundamental role in the energy balance of the body, regulating the metabolism of carbohydrates and lipids.

The side effects of metformin are quite common and include nausea, vomiting, diarrhoea, constipation, flatulence and, more rarely, lactic acidosis.

Other oral antidiabetics used in the treatment of type II diabetes include sulfonylureas, glinides, glitazones, DPP-4 enzyme inhibitors, intestinal alpha-glucosidase inhibitors, SGLT-2 renal glucose transporter inhibitors.

All of these drugs have almost the same side effects as metformin, some of which may also cause weight gain.

NAFLD ("Non-alcoholic Fatty Liver disease") is the most common liver disease in Western countries, with a sharp increase in recent years in developing countries as well. It is a disease closely related to obesity and insulin resistance and frequently present in patients with diabetes who are more likely to develop NASH ("Non-Alcoholic-Steatohepatitis") and cirrhosis of the liver. The most important therapeutic strategy for this disease is weight loss obtained through diet and physical activity. The drugs used include antidiabetic drugs (pioglitazone) and antiobesity drugs, including endocannabinoid receptor antagonists, but characterized by CNS side effects (depression and psychiatric disorders).

Considering the multifactorial etiology of cardiovascular disease, many patients require polytherapies, often consisting of antihypertensive, antidiabetic, and lipid-lowering drugs. This can more often lead to the onset of side effects and the abandonment of therapies.

The role of lifestyle (proper nutrition and physical activity) in cardiovascular prevention and in the fight against risk factors is known.

The use of so-called "nutraceuticals", foods or substances of plant or natural origin which are concentrated sources of nutrients or promote the physiological functions of the body is a useful tool, as part of a proper lifestyle.

Nutraceuticals can play a role in reducing disease risk factors, helping to improve the therapeutic response of patients, also potentially delaying or cancelling the need to resort to the use of pharmacological therapies.

The preparation object of the invention (herein defined as compound E) is characterized in that it has a standardized flavanones content of at least 40%, preferably 45%, by weight with respect to the total weight of the composition, preferably said composition consists of two or three extracts mixed or combined and/orpreferably the flavanones content is not more than 60% by weight with respect to the total weight of the composition, more preferably the composition comprises 35-40% w/w of non-HMG flavanones (3-Hydroxy-3-methyl-glutaryl) and at least 5% w/w of HMG flavanones, still more preferably the non-HMG flavanones and HMG flavanones content is 38% + at least 7%,

respectively.In fact, as will be shown below with particular examples, preparations as defined above exhibit higher activity than other ratios (e.g., higher HMG flavanones (A) content or higher non-HMG flavanones (B) content).

The above-described proportions in HMG and non-HMG flavanones allow to obtain a synergistic and broad spectrum effect of the preparation containing them with respect to preparations with higher amounts of HMG flavanones or higher amounts of non-HMG flavanones.

In fact, in the studies carried out, the results of A plus the results of B, taken individually, are lower than the results of E (= A+B giving 38+7%).

Therefore, the composition of the invention has a synergistic effect with respect to the individual components used both in HMG-CoA reductase inhibition tests and in AMPK activation measurement tests. The composition of the invention also has superior activity with respect to compositions characterized by different amounts of HMG and non-HMG flavanones in HMG-CoA reductase inhibition tests. These effects support the efficacy of the present composition in the prevention and/or treatment of cardiometabolic diseases, dyslipidaemias, insulin resistance, type II diabetes, NAFLD (non-alcoholic liver steatosis)/NASH (non-alcoholic steatohepatitis), hypertension, obesity, visceral fat, or to counteract disorders/risk factors typical of obesity and cardiovascular diseases, such as glycemia, triglycerides, cholesterol.

In another aspect of the invention, the preparation object of the invention (herein defined as compound F) is a preparation in micronized form, characterized in that it has a standardized flavanones content of at least 40% by weight with respect to the total weight of the composition, preferably the flavanones content is not more than 60% by weight with respect to the total weight of the composition, more preferably the composition comprises 35-40% w/w of non-HMG flavanones (3-Hydroxy-3-methyl-glutaryl) and at least 5% w/w of HMG flavanones, still more preferably the non-HMG flavanones and HMG flavanones content is 35% + at least 5%, respectively.

Indeed, as will be shown below, preparations as defined above exhibit superior activity with respect to similar non-micronized preparations.

In fact, in the studies carried out, the compound in micronized form of the invention showed a higher % of inhibition of HMG-CoA reductase with respect to that obtained with a similar non-micronized compound. The compositions of the invention also have higher efficacy with respect to compounds comprising higher amounts of HMG flavanones and/or higher amounts of non-HMG flavanones.

Therefore, an object of the present invention is a composition comprising or consisting of one or more plant extracts and comprising a flavanones content of at least 40% by weight with respect to the total weight of the composition.

Preferably said composition consists of two or three extracts. Preferably the flavanones content is at least 40, 41, 42, 43, 44, 45, 46, 47, 48, 49, 50 % w/w, preferably it is at least or equal to 40% w/w or 45%w/w.

Preferably, the flavanones content does not exceed 50, 51, 52, 53, 54, 55, 56, 57, 58, 59 or 60%, more preferably 60%, by weight with respect to the total weight of the composition.

The composition according to the invention is preferably in micronized form.

The composition according to the invention is preferably suitable for pharmaceutical use and/or as a dietary supplement.

Preferably, the composition of the invention comprises:

a) 35-40 % w/w of non-HMG flavanones (3-hydroxy-3-methyl-glutaryl) and
b) at least 5% w/w of HMG flavanones,

preferably the HMG flavanones content does not exceed 14% w/w, preferably it is 5 or 7%, preferably said composition is in micronized form.

Preferably the non-HMG flavanones content is 35% w/w and/or the HMG flavanones content is 5% w/w. In this case the composition is preferably in micronized form.

In an aspect of the invention, the flavanones content is at least 45% by weight with respect to the total weight of the composition. Preferably, the composition comprises:

a) 35-40 % w/w of non-HMG flavanones (3-hydroxy-3-methyl-glutaryl) and
b) at least 7% w/w of HMG flavanones.

The HMG flavanones content is preferably at least 5, 6, 7, 8, 9, 10, 11, 12, 13, 14% w/w, preferably it is equal to 5% or 7% w/w.

The HMG flavanones content is preferably about 5, 6, 7, 8, 9, 10, 10, 11, 12, 13, 14% w/w, preferably it is equal to 5 or 7% w/w.

Preferably the HMG flavanones content does not exceed 14% w/w, more preferably it is equal to 7%w/w.

Preferably the HMG flavanones content does not exceed 5, 6, 7, 8, 9, 10, 11, 12, 13, 14% w/w, more preferably it is equal to 5, 6, 7, 8, 9 or 10% w/w.

Preferably in the composition the non-HMG flavanones content is equal to 35%, 36%, 37%, 38%, 39%, 40%,

more preferably to 35 or 38% w/w. In the composition, preferably the HMG flavanones content does not exceed 14% w/w, more preferably it does not exceed 13%, or 12%, or 11% or 10% or 9% or 8% or 7% or 6% or 5% w/w.

In the composition, preferably the HMG flavanones content is equal to 7% w/w.

In a preferred embodiment of the invention in the composition of the invention the HMG flavanones content is 7% and the non-HMG flavanones content is 38% w/w.

In a preferred embodiment in the composition of the invention the HMG flavanones content is 5% and the non-HMG flavanones content is 35% w/w and the composition is in micronized form.

Preferably said extracts derive from fruits or parts thereof, more preferably from different fruits (or parts thereof).

Preferably the plant extract is selected from the group consisting of: bergamot extract (Citrus Bergamia Risso et Poiteau), pomelo extract (Citrus Maxima), grapefruit extract (Citrus x Paradisi), bitter orange extract (Citrus aurantium L. var. amara) or parts or fractions thereof or combinations or mixtures thereof.

Preferably the plant extract derives from (or is) juice and/or skins and/or "pastazzo", or from (or is) a mixture thereof.

Preferably, the composition as described above comprises or consists of:

- a first extract comprising a percentage of flavanones comprised in the following range: non-HMG flavanones 31-35% and HMG flavanones 5-11% or 12-15% by weight on the weight of the extract and of
- a second extract comprising a percentage of flavanones comprised in the following range: non-HMG flavanones 35-45% or 45-50% and HMG flavanones 0-5% by weight on the weight of the extract.
  Preferably, the composition consists of at least two extracts each having a known percentage of non-HMG flavanones and HMG flavanones, mixed or combined to obtain a composition characterized by a non-HMG flavanones content of 38% w/w and an HMG flavanones content of at least or equal to 7% w/w.

Preferably, the composition consists of at least two extracts each having a known percentage of non-HMG flavanones and HMG flavanones, mixed or combined to obtain a composition characterized by a non-HMG flavanones content of 35% w/w and an HMG flavanones content of at least or equal to 5% w/w. In this case, the composition is preferably micronized.

In the composition of the invention, preferably the non-HMG flavanones are selected from the group consisting of: Naringin, Neoeriotricin, Neoesperidin, or combinations thereof.

In the composition of the invention, the HMG flavanones are preferably selected from the group consisting of: Brutieridin, Melitidin, or combinations thereof.

The composition according to the invention is preferably in solid, semi-solid or liquid form.

The composition can be combined with anti-dyslipidemic and/or lipid-lowering agents and/or with other ingredients suitable for food or pharmaceutical use, such as one or more vitamins and/or minerals and/or enzymes and/or proteins and/or other plant extracts.

A further object of the invention is a pharmaceutical composition comprising the composition according to the invention and at least one pharmaceutically acceptable excipient and/or vehicle.

Another object of the invention is a dietary supplement comprising the composition as defined above.

In an embodiment, the invention provides a medical device (according to Italian Legislative Decree no. 46 of 24 February 1997) comprising the composition of the invention in the form of a preparation for oral intake comprising the composition as defined herein.

A further object of the invention is a composition or pharmaceutical composition or dietary supplement as defined above for medical use, preferably for use in the prevention and/or treatment of cardiometabolic diseases, dyslipidaemias, insulin resistance, type II diabetes, NAFLD/NASH, hypertension, obesity, visceral fat, to counteract disorders/risk factors typical of obesity, and cardiovascular diseases, such as glycemia, triglycerides, cholesterol.

A further object of the invention is the non-therapeutic use of the composition according to the invention or of the dietary supplement as defined above in the nutraceutical sector or as a basic ingredient in dietary supplements or drug preparations. In this embodiment preferably the composition is not in combination with anti-dyslipidemic and/or lipid-lowering agents.

The present composition is capable of inhibiting HMG-CoA reductase and/or increasing the expression and/or activity of AMPK (adenosine monophosphate-activated protein kinase). Such inhibition or increase is greater with respect to the inhibition or increase obtained with the individual extracts of the composition. A synergistic effect is thus obtained with the present composition. In the context of the present invention the w/w percentages (% w/w) are to be understood as percentages by weight on (or with respect to) the total weight of the composition (or of the compound), unless otherwise or differently stated.

When the composition of the invention consists of two or three extracts, they are understood as mixed or combined.

In the context of the present invention flavanones are intended as derivatives or as part of the plant extract(s).

Preferably, said composition or pharmaceutical composition or dietary supplement is administered orally, preferably once or twice a day.

Preferably the composition of the invention is for use as a pharmaceutical composition or dietary supplement or medical device. Accordingly, an object of the invention is the use of the composition of the invention as a pharmaceutical composition, dietary supplement or medical device.

In the context of the present invention the terms "preparation" or "composition" are used interchangeably. The term "composition" also comprises the terms "pharmaceutical composition" or "dietary supplement" or "medical device" as defined above.

The composition according to the present invention can be prepared by any method known to those skilled in the art, for example by mixing plant extracts (e.g., of bergamot) obtained from different matrices: juice and/or "pastazzo" (residues of skins, pulp and seeds after pressing the fruit and eliminating epicarp) and/or albedo with different extraction methods known to the skilled in the art (e.g., resins and membranes, KOH, supercritical $EtOH/H_2O/CO_2$); or mixing different extracts of different citrus fruits (e.g., bergamot and/or grapefruit and/or chinotto and/or bitter orange and/or pomelo or combinations thereof).

Preferably the plant extract is juice, for example obtained by pressing ripe fruits, after eliminating the epicarp. The juice obtained by squeezing can be subjected to an enzymatic process for the elimination of pectins. The liquid thus obtained is preferably subjected to a first membrane concentration process. The eluate is then preferably subjected to passage in adsorbent resins. The retentate of the resin is preferably washed with a mixture of water and ethanol and the obtained liquid is preferably subjected to solvent removal and a new membrane or heat concentration process. Finally, the product is preferably dried by spray-drying. Preferably the extract is obtained from the "pastazzo" (pulp residues, seed and albedo residues). The compound is preferably processed with the addition of water and enzymes and preferably subjected to depolpation and preferably to a subsequent ultrafiltration step. The filtration permeate is preferably passed in adsorbent resins. The resin retentate is preferably brought into solution by slightly alkaline aqueous solvent ($H_2O/KOH$). Finally, the product obtained is preferably subjected to a further process of membrane concentration, neutralization and elimination of the solvent and finally preferably dried by spray-drying.

Preferably the extract is a liquid obtained after eliminating the skins (epicarp) from the ripe fruits. The skins are preferably pressed in the presence of water and the obtained extract, after removal of the solid residues, is preferably passed in adsorbent resins. After washing the resins, preferably carried out with water or a mixture of water and ethanol, or alkaline solution $H_2O/KOH$, the flavonoid-rich resin retentate passes in solution and is preferably subsequently concentrated by membrane process (ultrafiltration, nanofiltration, reverse osmosis) or heat-concentration process, with elimination of the solvent. Finally, the product is preferably dried (by spray drying or lyophilization).

In the context of the present invention, "38 + 7" or "38% + 7%" or "38%w/w + 7%w/w" refers to the composition comprising 38% w/w non-HMG (3-Hydroxy-3-methyl-glutaryl) flavanones and at least 7% or 7% w/w HMG flavanones.

In the context of the present invention, "35 + 5" or "35% + 5%" or "35%w/w + 5%w/w" refers to the composition comprising 35% w/w non-HMG (3-Hydroxy-3-methyl-glutaryl) flavanones and at least 5% or 5% w/w HMG flavanones.

In the context of the present invention the term comprising also includes the term consisting of or characterized by.

"Micronized form" means that the powder particles present in the preparation have a diameter comprised between 0.1 and 20 $\mu$m.

The micronized form is obtained by a micronization process, carried out by injecting the material to be micronized into a high-pressure grinding chamber, operating with a gaseous fluid (compressed air or nitrogen).

The reduction in particle size occurs due to the very high velocity shocks which occur between the material particles themselves for the high-pressure passage of the fluid. The process is performed similarly to what is described in Chaumeil J. C. (1998). Micronization: a method of improving the bioavailability of poorly soluble drugs. Methods and findings in experimental and clinical pharmacology, 20(3), 211-215.

Said composition is preferably characterized in that said extracts or the composition itself is dehydrated or lyophilized or dried or in powder form.

Even more preferably said composition is characterized in that said extracts are dehydrated in the form of micronized, lyophilized or granulated particles.

The pharmaceutical composition according to the invention or the composition or the dietary supplement or the medical device according to the invention can be administered in the form of tablets, capsules, oral preparations, powders, granules, pills, injectables, or infusable liquid solutions, suspensions, suppositories, preparation for inhalation.

Tablets and capsules for oral administration are normally presented in unit dose form and contain conventional excipients such as binders, fillers (including cellulose, mannitol, lactose), diluents, tablet agents, lubricants (including magnesium stearate), detergents, disintegrants (e.g., polyvinylpyrrolidone and starch derivatives such as sodium starch glycolate), colouring agents, flavouring agents and wetting agents (e.g., sodium lauryl sulphate).

Liquid preparations can for example be in aqueous form in oily suspension, solutions, emulsions, syrups or can be presented as a dry product for reconstitution with water. Liquid preparations can contain conventional additives, such as

suspension agents, emulsifying agents, non-aqueous vehicles.

A reference for the formulations is the book by Remington ("Remington: The Science and Practice of Pharmacy", Lippincott Williams & Wilkins, 2000).

The composition, pharmaceutical composition or dietary supplement according to the invention can be administered in a single dosage containing all the components or as separate (simultaneous or sequential) compositions of the individual components. The composition, pharmaceutical composition or dietary supplement can be administered in combination with active ingredients which can be separately formulated in single ingredient preparations of one of the forms described above and then administered as combined preparations which are given at the same time or at different times, or can be formulated together in the same preparation.

Those skilled in the art will choose the form of administration and the effective dosages by selecting suitable diluents, adjuvants and/or excipients.

Preferably, said composition, pharmaceutical composition or dietary supplement is in solid form, for example a tablet, a hard capsule, an animal or vegetable gelatin soft capsule, a powder, a syrup, a cachet, a lozenge, a pill or a tablet, sachet or stick pack, or in liquid form, for example an oral spray.

Preferably, said pharmaceutically acceptable excipient or diluent or carrier is selected from the group consisting of: calcium phosphate, dicalcium phosphate, microcrystalline cellulose, magnesium stearate, silicon dioxide, sucrose, gum arabic, corn starch, medium-chain triglycerides, tricalcium phosphate, cross-linked sodium carboxymethylcellulose, hydroxypropylmethylcellulose, polyethylene glycol, titanium dioxide, polyvinylpyrrolidone, talc, erythritol, xylitol, steviol glycosides and sucralose.

The pharmaceutical composition can be chosen based on the treatment to be performed.

Preferably, said pharmaceutical composition is administered orally. Preferably, said pharmaceutical composition is administered once or twice a day.

The composition object of the present invention, preferably in the form of dry extracts, can be used in the nutraceutical field for example in the form of tablets by adding at least one excipient, or can be encapsulated in vegetable gelatin capsules with the addition of at least one pharmaceutically acceptable excipient.

The present composition can be mixed or combined with other active ingredients and/or substances of plant or natural or synthetic origin, and/or vitamins and/or minerals and/or with the addition of excipients or combinations thereof.

Examples of natural substances or ingredients suitable for food or pharmaceutical use are: monacolin k, berberine, artichoke, garlic, polycosanols, Olea Europaea, plant sterols or stanols, silymarin, green tea extracts, monounsaturated or polyunsaturated fatty acids or chitosan or combinations thereof.

Examples of vitamins are: vitamin C, E or D or combinations thereof. Examples of minerals/trace elements are: chromium, copper or manganese or combinations thereof. Food supplement also refers to a food product or a beverage.

It is a further object of the present invention a dietary supplement or a food product or a beverage comprising the composition as defined above and at least one excipient or diluent, and optionally, a further agent.

Preferably, said dietary supplement or food product or beverage is for use in the prevention, control and/or treatment of cardiometabolic diseases, dyslipidaemias, insulin resistance, type II diabetes, NAFLD/NASH, hypertension, obesity, visceral fat, to counteract disorders/risk factors typical of obesity and cardiovascular diseases, such as glycemia, triglycerides, cholesterol.

Preferably, said excipient or diluent is selected from the group consisting of: calcium phosphate, dicalcium phosphate, microcrystalline cellulose, magnesium stearate, silicon dioxide, sucrose, gum arabic, corn starch, medium-chain triglycerides, tricalcium phosphate, cross-linked sodium carboxymethylcellulose, hydroxypropylmethylcellulose, polyethylene glycol, titanium dioxide, polyvinylpyrrolidone, talc, erythritol, xylitol, steviol glycosides and sucralose.

Preferably, said dietary supplement or food product or beverage is administered orally. Preferably, said dietary supplement or food product or beverage is administered once or twice a day. Preferably, said dietary supplement or food product or beverage is in the form of a tablet, a hard capsule, a soft capsule of vegetable or animal gelatin, a powder, a syrup, a cachet, a pill, a tablet, a lozenge, a dietary supplement, an edible bar, or an edible snack. In particular, said dietary supplement can be any type of dietary supplement.

The amounts of flavanones are measured by any technique known to those skilled in the art, for example by HPLC.

The present pharmaceutical composition or dietary supplement preferably comprises an amount from 200 mg to 500 mg, more preferably 200 mg to 400 mg, more preferably of about 200, 400 or 500 mg of the composition according to the invention.

Preferably the present composition further comprises vitamin E and/or plant sterols (phytosterols) and/or astaxanthin and/or olea europaea extract.

The invention will be illustrated by the following non-limiting examples.

**EXAMPLES**

**Description of the production process of extracts A, B, C**

**Extract A** (juice obtained by squeezing ripe fruits, after eliminating the epicarp).

The juice obtained by squeezing is subjected to an enzymatic process to eliminate the pectins. The liquid thus obtained is subjected to a first membrane concentration process. The eluate is then subjected to passage in adsorbent resins. The retentate of the resin is washed with a mixture of water and ethanol and the obtained liquid is subjected to solvent removal and a new membrane or heat concentration process. Finally, the product is dried by spray-drying.

**Extract B** (product obtained by processing "pastazzo" (remnants of pulp, seeds and albedo)).

The compound is processed with the addition of water and enzymes and subjected to depolpation and to a subsequent ultrafiltration step. The filtration permeate is passed in adsorbent resins. The resin retentate is brought into solution by slightly alkaline aqueous solvent ($H_2O$/KOH). Finally, the product obtained is subjected to a further process of membrane concentration, neutralization and elimination of the solvent and finally drying by spray drying.

**Extract C** - optional (liquid obtained after eliminating the skins (epicarp) from the ripe fruits).

The skins are pressed in the presence of water and the obtained extract, after removal of the solid residues, is passed in adsorbent resins. After washing the resins, carried out with water or a mixture of water and ethanol, or alkaline solution $H_2O$/KOH, the flavonoid-rich resin retentate passes in solution and is subsequently concentrated by membrane process (ultrafiltration, nanofiltration, reverse osmosis) or heat-concentration process, with elimination of the solvent. Finally, the product is dried (by spray drying or lyophilization).

**Examples of mixing to obtain the ratio 38+7:**

To obtain the compound object of the present invention it is possible to mix at least two extracts, possibly adding a third extract to adjust the titre.

To calculate the percentage of extract A and extract B required to obtain compound E, the following formulae are applied:

$$xA + (1-x)B = 38$$

$$YA + (1-y)B = 7$$

Where A = % w/w of flavanones in extract A and B = % w/w of flavanones in extract B

In order to be useful and acceptable for use in the mixing and obtainment of compound E, the 2 extracts A and B must have a percentage of flavanones comprised in the following range:
Juice extract (A): 31-35% w/w Non-HMG and 12-15% w/w HMG flavanones Extracted from pressing skins or from "pastazzo" (B): 45-50% Non-HMG and 0-5% HMG flavanones

**Example 1:**

Non-HMG flavanones content of starting compounds:

Compound A = 31% w/w
Compound B = 45% w/w

HMG flavanones content of the starting compounds:

Compound A = 12% w/w
Compound B = 2% w/w

Applying the formula described above, it emerges that the necessary percentage of each compound A and B is 50%.

Therefore, A and B are mixed in equal parts, resulting in a compound with 38% w/w of non-HMG flavanones and 7% w/w of HMG flavanones.

**Example 2:**

Non-HMG flavanones content of the starting compounds:

Compound A = 35% w/w
Compound B = 45% w/w

HMG flavanones content of the starting compounds:

Compound A = 15% w/w
Compound B = 1% w/w

By applying the formulae, the amount necessary to obtain 38% w/w of non-HMG flavanones and a minimum of 7% w/w of HMG flavanones is obtained.
In this case, 10.8% w/w of HMG flavanones and 38% w/w of non-HMG flavanones are obtained by mixing 70% of A and 30% of B.

**Example 3:**

Non-HMG flavanones content of the starting compounds:

Compound A = 32% w/w
Compound B = 48% w/w

HMG flavanones content of the starting compounds:

Compound A = 12% w/w
Compound B = 3% w/w

By applying the formulae, the amount necessary to obtain 38% w/w of non-HMG flavanones and a minimum of 7% w/w of HMG flavanones is obtained.
In this case we obtain 8.6% w/w of HMG flavanones and 38% w/w of non-HMG flavanones by mixing 62.5% A and 37.5% B.

**Example 4:**

The preparation object of the invention has also been obtained by mixing extracts of different citrus fruits, for example:
a Pomelo extract (Citrus Maxima tomentosa) containing HMG in % equal to 2-10% w/w, a Grapefruit extract (Citrus Paradisi) containing minimum 45% w/w naringin.
These different citrus extracts can be mixed with each other and/or in combination with a suitable bergamot extract to obtain the desired percentages of HMG and non-HMG flavanones, applying the same formula mentioned above in example 4.

Non-HMG flavanones content of the starting compounds:

Pomelo (Compound A) = 32% w/w
Grapefruit (Compound B) = 48% w/w

HMG flavanones content of the starting compounds:

Pomelo (Compound A) = 9% w/w
Grapefruit (Compound B) = 3% w/w

By mixing 62% of Pomelo and 37% of Grapefruit (B) a compound with 38% w/w of non-HMG flavanones and 6.75% w/w of HMG flavanones is obtained.
At this point to obtain the desired titre the compound must be mixed with another suitable extract.
The obtained compound is mixed with a bergamot juice extract, rich in HMG flavanones and poorer in non-HMG flavanones.

Bergamot juice extract (C)
Non-HMG content = 33% w/w
HMG content = 14% w/w

By mixing 96% of the first compound and 4% of the second, the extract at 38% w/w + 7% w/w is obtained.
The compound obtained by mixing different citrus extracts, with its titre in HMG and non-HMG flavanones, can also be mixed by applying the same formula described above to a bergamot extract suitable by titre, to obtain the desired result, i.e., 38 + 7.

Final preparation

The final preparation is a fine powder, of variable colour from pale yellow to green, moderately soluble in water, with a pungent smell, characteristic of citrus and bitter taste. Such a preparation can be used in the preparation of solid pharmaceutical forms such as tablets, capsules, sachets, stick packs or liquids, also in mixing with other active ingredients of plant or natural origin or synthetic, or vitamins or minerals and with the addition of excipients.

Formulation examples:

Capsule containing 200 mg of compound E + other active ingredients + excipients

Ingredients:

Compound E 200 mg
Vitamin E 60 mg
Olea europaea extract 500 mg

Excipients and casing:

Hydroxypropyl methylcellulose capsule
Magnesium stearate as needed
Silicon dioxide as needed

Tablet containing 400 mg of compound E + other active ingredients + excipients

Ingredients:

Compound E 400 mg
Plant sterols (Phytosterols) 400 mg
Astaxanthin 0.5 mg

Excipients as needed:

Bulking agents: cellulose, dicalcium phosphate
Anti-caking agents: Fatty acid magnesium salts, silicon dioxide
Coating agents: hydroxypropyl methyl cellulose, calcium carbonate

Capsule containing 500 mg of compound E + excipients

Ingredients:
Compound E 500 mg
Excipients and casing:

Hydroxypropyl methylcellulose capsule
Maltodextrins as needed

**In vitro tests conducted to measure the activity of the preparation and synergistic effect of the mixture object of the invention with respect to the starting compounds**

*HMGcoA reductase inhibition test*

The purpose of the test is to evaluate the hypocholesterolemic activity of the test samples by determining the inhibition of the enzyme 3-hydroxy-3-methylglutaryl-CoA reductase (HMG-CoA reductase).

**Materials and Methods**

The test for the HMG-CoA reductase enzyme inhibitory capacity was performed using the special kit (HMG-CoA Reductase Assay Kit, Catalogue Number CS1090, Sigma-Aldrich) and related experimental protocol.
The experiments were carried out in triplicate.

**Experimental protocol**

5 mL of buffer were added to 50 mg of the test sample and the solution obtained was then suitably filtered and used for the test.
A 100 μM Pravastatin solution (0.042 mg/mL, Sigma Aldrich) was used as a positive control.
Below: A = extract from juice and B = extract from bergamot skins E = A+B = 38% non-HMG flavanones + ≥ 7% HMG flavanones.
The samples were prepared according to the scheme shown in *Table 1.*

*Table 1*

| SAMPLE | BUFFER | PRAVASTATIN | Sample A | Sample B | Sample C | NAPDH | HMG-COA | HMG-COA REDUCTASE |
|---|---|---|---|---|---|---|---|---|
| White | 184 μl | - | - | - | - | 4 μl | 12 μl | - |
| Control | 182 μl | - | - | - | - | 4 μl | 12 μl | 2 μl |
| Positive control (Inhibition) | 181 μl | 1 μl | - | - | - | 4 μl | 12 μl | 2 μl |
| Tested sample | 181 μl | - | 1 μl | 1 μl | 1 μl | 4 μl | 12 μl | 2 μl |

The samples thus prepared were stirred for 10 seconds. Absorbance was then measured at 340 nm for 10 minutes while maintaining the samples at 37°C.
The ability to inhibit enzyme activity was then expressed as percent inhibition and calculated according to the following Equation (1):

$$\%\,Hinibition = \left(\frac{\Delta A_{Control} - \Delta A_{sample}}{\Delta A_{Control}}\right) x\ 100\%$$

where the value of ΔA is given by the difference between the absorbance recorded at the start time and that recorded after 10 minutes for the control and for the sample respectively.

**Results**

The test is based on the spectrophotometric measurement of the decrease in absorbance at 340 nm due to a decrease in the concentration of NADPH following its oxidation by the HMG-CoA reductase enzyme in the presence of the HMG-CoA substrate. The reaction occurs according to the following scheme:

HMG-CoA + 2NADPH + 2H+ $\rightarrow$ mevalonate + 2NADP+ + CoA-SH

The obtained results were expressed as percentage of inhibition, calculated in accordance with Equation (1) and shown in *Table 2.*

*Table 2*

| SAMPLE | INHIBITION OF HMG-CoA REDUCTASE (%) |
|---|---|
| Positive control (Pravastatin 0.042 mg/ml) | 99.6 ± 1.1 |
| Sample A (10 mg/ml) | 40.8 ± 0.7 |
| Sample B (10 mg/ml) | 9.5 ± 0.9 |
| Sample E (10 mg/ml) | 55.6 ±0.6 |

**Evaluation of the effects of the tested compounds on AMPK phosphorylation**

AMPK (AMP-activated protein kinase) is a protein of the serine-threonine-kinase family, considered the main cellular energy sensor. Its activation is favoured by conditions which involve energy depletion and which induce an increase in the AMP/ATP ratio.

Under these conditions, AMPK inhibits anabolic processes and activates catabolic processes to restore ATP reserves.

AMPK is activated by several drug and non-pharmacological substances, including metformin and resveratrol.

The activation of AMPK is related to favourable metabolic effects for the body such as inhibition of proliferation of cancer cells, increase in beta-oxidation of fatty acids and decrease in the synthesis thereof, increase in glycolysis and inhibition of gluconeogenesis, activation of GLUT-4 channels of glucose, with an increase in cellular uptake of the same.

**Materials and Methods**

AMPK activation was measured in cultured liver cells (HepG2 (Catalogue Number 85011430, Sigma-Aldrich) by means of commercial kits for the quantitative measurement of AMPK phosphorylation (cell-based ELISA). In detail, it was chosen to use the AMPK Phosphorylation Assay Kit (Catalogue Number LS-K268-100) according to the manufacturer's protocol. This ELISA test measures phosphorylated AMPK in whole cells and normalizes the signal on the total protein content.

In particular, 1-3x10^4 HepG2 cells are seeded in black 96-well plates and incubated overnight at 37°C. Cell-free culture media were distributed in 3 wells for Protein White.

The tested extracts (A, B, E) were added at two different concentrations (1 and 5 mg/ml) in triplicate for 2 hours. The medium alone was added to the cells as a control.

At the end of treatment, the cells were fixed in 4% formaldehyde, washed and incubated with the primary antibody against phosphorylated AMPK (pAMPK-Thr-172). A second conjugated HRP antibody was subsequently added.

Fluorescence was measured at $\lambda$ex/em = 530/585 nm for phosphorylated AMPK (pAMPK) and at $\lambda$ex/em =360/450 nm for total protein content. Relative pAMPK levels, calculated with respect to the cells treated with the control, were calculated as follows:

$$\text{pAMPK Levels} = \frac{(\Delta F_{pAMPK}/\Delta F_{prot})}{(\Delta F_{pAMPK}/\Delta F_{prot})_{Ctrl}}$$

Where:

$$\Delta F_{pAMPK} = F\text{sample}_{pAMPK} - F\text{Blank}_{pAMPK}$$

$$\Delta F_{pro} = F\text{sample}_{prot} - F\text{Blank}_{prot}$$

$$(\Delta F_{pAMPK}/\Delta F_{prot})_{Ctrl} = \text{control reference value (e.g., untreated cells)}$$

**Statistical analysis**

Statistical analysis was performed by Statview Statistical Package, Version 5.0.1 (SAS Institute, Abacus Concept, Inc., Berkeley, California), the Microsoft Excel 2010 calculation program and the GraphPad Prism 6 statistical package.

For data concerning the evaluation of AMPK expression, Student's t-test was applied for unpaired data.

P values < 0.01 with respect to the control were considered statistically significant.

The data are expressed as mean ± SE (standard error).

**Results**

AMPK expression is statistically significantly increased in all the tested samples with respect to the control.

However, it is evident that the greatest increase in expression is evident in sample E with respect to A and B (Table 3).

*Table 3*

| SAMPLE | AMPK EXPRESSION (%) at 6h |
|---|---|
| Control | 1.1 ± 0.1 |
| Sample A (10 $\mu M$) | 31.2 ± 1.7* |
| Sample B (10 $\mu M$) | 52.8 ± 1.9* |
| Sample E (10 $\mu M$) | 84.7 ±1.6* |
| *p<0.01 vs control | |

**Synergistic effect**

The synergistic effect was evaluated according to the method described by S.R. Colby in "Calculation of the synergistic and antagonistic responses of herbicide combinations" Weeds, 1967.

The synergy factor was calculated for each compound. A factor >1 indicates the existence of a synergistic effect. A factor <1 indicates the existence of an antagonistic effect.

The formula applied in the calculations is as follows:

$$\textbf{Expected efficacy rate} = A+B - (A*B/100)$$

**Synergy factor (FS)** = (1* observed efficacy rate (%)) / expected efficacy rate (%)

Calculation of the synergy factor for A+B = E where A = extract from juice and B= extract from bergamot skins E= A+B =38% non-HMG flavanones + ≥ 7% HMG flavanones.

| | Observed efficacy rate (%) | | | Expected efficacy rate for E | Synergy factor |
|---|---|---|---|---|---|
| | A | B | E | | |
| **Inhibition of HMG- CoA reductase** | 40.8 | 9.5 | 55.6 | 46.4 % | 1.19 |
| **AMPK increase** | 31.2 | 52.8 | 84.7 | 67.6 % | 1.25 |

**HMG-CoA reductase test Compound E versus compound D (42.77% non-HMG + 5.99% HMG flavanones)**

The preparation described in the study by Ballistreri et al., 2020, "Evaluation of lipid and cholesterol-lowering

effect of bioflavonoids from bergamot extract", Natural Product Research, hereinafter referred to as "sample D" or "compound D" is an extract from bergamot juice (Bergastat ® batch no. 19/064) containing 54.96 g/100 g of total flavanones for a total of non-HMG flavanones (calculated as the sum of neoeriotricin, naringin, neoesperidin) of 42.77% and HMG flavanones (as the sum of brutieridin and melitidin) of 5.99%.

In order to compare the biological effects of compound D with respect to compound E (sample E), an in vitro study on HMG-CoA reductase inhibition was conducted as in the above example, this time using a concentration of compound E equal to 0.85 mg/mL (850 μg/mL) or identical to the concentration of compound D used in the test described in the above study (Ballistreri et al., 2020). A solution of Pravastatin at the concentration of 0.01 mg/mL (10 μg/mL) was used as positive control.

*Results:*

In the Ballistreri et al. study, compound D at a concentration of 0.85 mg/mL achieved a % HMG-CoA reductase inhibition of 85%.

In the experiment with compound E, a result of 92% (±0.9) was obtained at a concentration of 0.85 mg/mL. The positive control showed a result equal to 99.8% (±0.7).

Therefore, not only it was obtained a result higher than compound D at the same concentration, but from this result it can be seen that a lower concentration allows to obtain higher results with respect to the concentration of 10 mg/mL.

**Examples of micronized preparations with a ratio of 35+5:**

To obtain the compound object of the present invention it is possible to mix at least two extracts, possibly adding a third extract to adjust the titre with the same modes described above.

The two extracts to be mixed must have a titre in HMG flavanones respectively between 5-11% for extract 1 and between 0-5% for extract 2 and a titre in non-HMG flavanones of 31-35% for extract 1 and 35-45% for extract 2.

**Preparation example**

A bergamot extract obtained by mixing an extract A (obtained from bergamot juice, as described above) and an extract C (obtained from processing bergamot skins) in the proportions 30% and 70% w/w, respectively, was prepared.

The starting extracts were characterized by an HMG flavanones content equal to 9% and 1% respectively and of non-HMG flavanones equal to 33% and 37% respectively.

Upon HPLC analysis, the preparation, defined as "compound F", was characterized by an HMG flavanones content of 5% and non-HMG flavanones content of 35%.

The preparation was subjected to a micronization process, which consists in reducing the powder particles from a diameter between 40 and 100 μm to a diameter between 0.1 and 20 μm.

Final preparation

The final preparation is a fine powder, of variable colour from pale yellow to green, moderately soluble in water, with a pungent smell, characteristic of citrus and bitter taste. Such a preparation can be used in the preparation of solid pharmaceutical forms such as tablets, capsules, sachets, stick packs or liquid pharmaceutical forms , also in mixing with other active ingredients of plant or natural origin or of synthesis, or vitamins or minerals and with the addition of excipients.

Formulation examples:

Capsule containing 200 mg of compound F + other active ingredients + excipients

Ingredients:

Compound F 200 mg
Vitamin E 60 mg
Olea europaea extract 500 mg

Excipients and casing:

Hydroxypropyl methylcellulose capsule
Magnesium stearate as needed
Silicon dioxide as needed

Tablet containing 400 mg of compound F + other active ingredients + excipients

Ingredients:

Compound F 400 mg
Plant sterols (Phytosterols) 400 mg
Astaxanthin 0.5 mg

Excipients as needed:

Bulking agents: cellulose, dicalcium phosphate
Anti-caking agents: Fatty acid magnesium salts, silicon dioxide
Coating agents: hydroxypropyl methyl cellulose, calcium carbonate

Capsule containing 500 mg of compound F + excipients Ingredients:

Compound F 500 mg
Excipients and casing:

Hydroxypropyl methylcellulose capsule
Maltodextrins as needed

**HMG-CoA reductase inhibition test**

To evaluate the effectiveness of the compound thus obtained, called "compound F", an HMG-CoA reductase inhibition test was carried out, according to the procedure described above. Compound F was tested at the concentration of 0.85 mg/mL and a Pravastatin solution at the concentration of 0.01 mg/mL was used as a positive control.

Compound F exhibited a % of inhibition of HMG-CoA reductase of 93% (±1.1), higher than that obtained with compound D as described in the study by Ballistreri et al. (i.e., 85%). The positive control result was 99.8% (±0.9).

Therefore, compound F, although containing a lower % of flavanones with respect to compound D, achieved higher in vitro results with respect thereto, and comparable to the results obtained with compound E.

## Claims

1. Composition consisting of one or more plant extracts and comprising a flavanones content of at least 40% by weight with respect to the total weight of the composition, said composition comprising:

   a) 35-40% w/w of non-HMG flavanones (3-hydroxy-3-methyl-glutaryl) and
   b) at least 5% w/w of HMG flavanones,

   preferably said composition consists of two or three extracts mixed or combined and/or preferably wherein the flavanones content does not exceed 60% by weight with respect to the total weight of the composition, preferably wherein said composition is in micronized form.

2. The composition according to claim 1 suitable for pharmaceutical use and/or as a dietary supplement.

3. The composition according to claim 1 or 2 wherein the HMG flavanones content does not exceed 14% w/w, preferably it is equal to 5% or 7%, preferably
   wherein said composition is in micronized form.

4. The composition according to any one of previous claims, wherein the non-HMG flavanones content is equal to 35% w/w, preferably wherein the HMG flavanones content is equal to 5% w/w.

5. The composition according to any one of previous claims, wherein the non-HMG flavanones content is equal to 35% w/w, the HMG flavanones content is equal to 5% w/w and the composition is in micronized form.

6. The composition according to any one of claims 1-3 wherein the flavanones content is of at least 45% by weight with respect to the total weight of the composition.

7. The composition according to claim 1, 2 or 6 comprising:

a) 35-40% w/w of non-HMG flavanones (3-hydroxy-3-methyl-glutaryl) and
b) at least 7% w/w of HMG flavanones,

preferably the HMG flavanones content does not exceed 14% w/w, preferably it is equal to 7%.

8. The composition according to claim 1, 2, 6 or 7, wherein the non-HMG flavanones content is equal to 38% w/w, preferably wherein the HMG flavanones content is equal to 7% w/w.

9. The composition according to any one of claims 1-2, or 6-8, wherein the non-HMG flavanones content is equal to 38% w/w, the HMG flavanones content is equal to 7% w/w.

10. The composition according to one of the preceding claims wherein the plant extract is selected from the group consisting of: bergamot extract (Citrus Bergamia Risso et Poiteau), pomelo extract (Citrus Maxima), grapefruit extract (Citrus x Paradisi), bitter orange extract (Citrus aurantium **L.** var. amara) or parts or fractions thereof or combinations or mixtures thereof, preferably by juice and/or peels and/or "pastazzo",

and/or wherein said composition consists of:

- a first extract comprising a flavanones percentage comprised within the following range: non-HMG flavanones 31-35% and HMG flavanones 5-11% or 12-15% by weight on the weight of the extract and by
- a second extract comprising a flavanones percentage comprised in the following range: non-HMG flavanones 35-45% or 45-50% and HMG flavanones 0-5% by weight on the weight of the extract

and/or wherein the non-HMG flavanones are selected from the group consisting of: Naringin, Neoeriotricin, Neoesperidin or combinations thereof and/or
the HMG flavanones are selected from the group consisting of: Brutieridin, Melitidin or combinations thereof and/or
wherein the composition is in solid, semi-solid or liquid form and/or in combination with antidislipidemic and/or lipid-lowering agents and/or with other ingredients suitable for food or pharmaceutical use, such as one or more vitamins, minerals, enzymes, proteins, and/or other plant extracts.

11. The composition according to any of previous claims consisting of at least two extracts each having a known percentage of non-HMG flavanones and HMG flavanones, mixed or combined as to obtain:

- a composition **characterized by** a non-HMG flavanones content of 35% w/w and a HMG flavanones content equal to 5% w/w wherein said composition is in micronized form or
- a composition **characterized by** a non-HMG flavanones content of 38% w/w and a HMG flavanones content equal to 7% w/w.

12. A pharmaceutical composition comprising the composition according to any of claims 1-11 and at least one pharmaceutically acceptable excipient and/or vehicle.

13. A dietary supplement comprising the composition according to any of claims 1-11.

14. The composition according to any of claims 1-11 or the pharmaceutical composition according to claim 12 or the dietary supplement according to claim 13 for medical use, preferably for use in preventing and/or treating cardio-metabolic diseases, dyslipidaemias, insulin resistance, type 2 diabetes, NAFLD (non-alcoholic hepatic steatosis)/-NASH (non-alcoholic steatohepatitis), hypertension, obesity, visceral fat, to counteract disorders/risk factors typical of obesity and cardiovascular diseases, such as glycemia, triglycerides, cholesterol.

15. Non-therapeutic use of the composition according to one of claims 1 to 11 or of the food supplement according to claim 13 as a basic ingredient in preparations of food supplements or drugs.

## Patentansprüche

1. Zusammensetzung, bestehend aus einem oder mehreren Pflanzenextrakten und umfassend einen Flavanon-Gehalt von mindestens 40 Gew.-%, bezogen auf das Gesamtgewicht der Zusammensetzung, wobei die Zusammensetzung umfasst:

   a) 35 - 40 % w/w Nicht-HMG-Flavanone (3-Hydroxy-3-methyl-glutaryl) und
   b) mindestens 5 % w/w HMG-Flavanone, wobei vorzugsweise die Zusammensetzung aus zwei oder drei gemischten oder kombinierten Extrakten besteht und/oder wobei vorzugsweise der Flavanon-Gehalt 60 Gew.-%, bezogen auf das Gesamtgewicht der Zusammensetzung, nicht übersteigt,

   wobei vorzugsweise die Zusammensetzung in mikronisierter Form vorliegt.

2. Zusammensetzung nach Anspruch 1, geeignet für pharmazeutische Verwendung und/oder als Nahrungsergänzungsmittel.

3. Zusammensetzung nach Anspruch 1 oder 2, wobei der Gehalt an HMG-Flavanonen 14 % w/w nicht übersteigt, wobei er vorzugsweise gleich 5 % oder 7 % ist, wobei vorzugsweise die Zusammensetzung in mikronisierter Form vorliegt.

4. Zusammensetzung nach einem der vorstehenden Ansprüche, wobei der Gehalt an Nicht-HMG-Flavanonen gleich 35 % w/w ist, wobei vorzugsweise der Gehalt an HMG-Flavanonen gleich 5 % w/w ist.

5. Zusammensetzung nach einem der vorstehenden Ansprüche, wobei der Gehalt an Nicht-HMG-Flavanonen gleich 35 % w/w ist, der Gehalt an HMG-Flavanonen gleich 5 % w/w ist und die Zusammensetzung in mikronisierter Form vorliegt.

6. Zusammensetzung nach einem der Ansprüche 1 bis 3, wobei der Flavanon-Gehalt mindestens 45 Gew.-%, bezogen auf das Gesamtgewicht der Zusammensetzung, beträgt.

7. Zusammensetzung nach Anspruch 1, 2 oder 6, umfassend:

   a) 35 - 40 % w/w Nicht-HMG-Flavanone (3-Hydroxy-3-methyl-glutaryl) und
   b) mindestens 7 % w/w HMG-Flavanone,

   wobei vorzugsweise der Gehalt an HMG-Flavanonen 14 % w/w nicht übersteigt, wobei er vorzugsweise gleich 7 % ist.

8. Zusammensetzung nach Anspruch 1, 2, 6 oder 7, wobei der Gehalt an Nicht-HMG-Flavanonen gleich 38 % w/w ist, wobei vorzugsweise der Gehalt an HMG-Flavanonen gleich 7 % w/w ist.

9. Zusammensetzung nach einem der Ansprüche 1 bis 2 oder 6 bis 8, wobei der Gehalt an Nicht-HMG-Flavanonen gleich 38 % w/w ist und der Gehalt an HMG-Flavanonen gleich 7 % w/w ist.

10. Zusammensetzung nach einem der vorstehenden Ansprüche, wobei der Pflanzenextrakt aus der Gruppe ausgewählt ist, bestehend aus: Bergamotte-Extrakt (Citrus bergamia Risso et Poiteau), Pomelo-Extrakt (Citrus maxima), Grapefruit-Extrakt (Citrus x Paradisi), Bitterorangen-Extrakt (Citrus aurantium L. var. amara) oder Teilen oder Fraktionen davon oder Kombinationen oder Mischungen davon, vorzugsweise aus Saft und/oder Schalen und/oder "Pastazzo",

    und/oder wobei die Zusammensetzung besteht aus:

    - einem ersten Extrakt, umfassend einen Flavanon-Prozentanteil im folgenden Bereich: Nicht-HMG-Flavanone 31 - 35 % und HMG-Flavanone 5 - 11 % oder 12 - 15 Gew.-%, bezogen auf das Gewicht des Extrakts, und
    - einem zweiten Extrakt, umfassend einen Flavanon-Prozentanteil im folgenden Bereich: Nicht-HMG-

Flavanone 35 - 45 % oder 45 - 50 % und HMG-Flavanone 0 - 5 Gew.-%, bezogen auf das Gewicht des Extrakts,

und/oder wobei die Nicht-HMG-Flavanone ausgewählt sind aus der Gruppe bestehend aus: Naringin, Neoeriotricin, Neoesperidin oder Kombinationen davon und/oder
die HMG-Flavanone ausgewählt sind aus der Gruppe bestehend aus: Brutieridin, Melitidin oder Kombinationen davon, und/oder
wobei die Zusammensetzung in fester, halbfester oder flüssiger Form und/oder in Kombination mit antidislipidämischen und/oder lipidsenkenden Mitteln und/oder mit anderen für den Lebensmittel- oder pharmazeutischen Gebrauch geeigneten Inhaltsstoffen vorliegt, wie beispielsweise einem oder mehreren Vitaminen, Mineralstoffen, Enzymen, Proteinen und/oder anderen Pflanzenextrakten.

**11.** Zusammensetzung nach einem der vorstehenden Ansprüche, bestehend aus mindestens zwei Extrakten, die jeweils einen bekannten Prozentanteil an Nicht-HMG-Flavanonen und HMG-Flavanonen aufweisen, gemischt oder kombiniert, um Folgendes zu erhalten:

- eine Zusammensetzung, **gekennzeichnet durch** einen Gehalt an Nicht-HMG-Flavanonen von 35 % w/w und einen Gehalt an HMG-Flavanonen von gleich 5 % w/w, wobei die Zusammensetzung in mikronisierter Form vorliegt, oder
- eine Zusammensetzung, **gekennzeichnet durch** einen Gehalt an Nicht-HMG-Flavanonen von 38 % w/w und einen Gehalt an HMG-Flavanonen von gleich 7 % w/w.

**12.** Pharmazeutische Zusammensetzung, umfassend die Zusammensetzung nach einem der Ansprüche 1 bis 11 und mindestens einen pharmazeutisch verträglichen Hilfsstoff und/oder Trägerstoff.

**13.** Nahrungsergänzungsmittel, umfassend die Zusammensetzung nach einem der Ansprüche 1 bis 11.

**14.** Zusammensetzung nach einem der Ansprüche 1 bis 11 oder pharmazeutische Zusammensetzung nach Anspruch 12 oder Nahrungsergänzungsmittel nach Anspruch 13 zur medizinischen Verwendung, vorzugsweise zur Verwendung bei der Vorbeugung und/oder Behandlung von kardiometabolischen Erkrankungen, Dyslipidämien, Insulinresistenz, Typ-2-Diabetes, NAFLD (nichtalkoholische Fettleber) / NASH (nichtalkoholische Steatohepatitis), Bluthochdruck, Adipositas, viszeralem Fett sowie zur Bekämpfung von für Adipositas und Herz-Kreislauf-Erkrankungen typischen Störungen/Risikofaktoren wie Glykämie, Triglyceride, Cholesterin.

**15.** Nichttherapeutische Verwendung der Zusammensetzung nach einem der Ansprüche 1 bis 11 oder des Nahrungsergänzungsmittels nach Anspruch 13 als Grundstoff in Zubereitungen von Nahrungsergänzungsmitteln oder Arzneimitteln.

## Revendications

**1.** Composition constituée d'un ou de plusieurs extraits végétaux et comprenant une teneur en flavanones d'au moins 40 % en poids par rapport au poids total de la composition, ladite composition comprenant :

a) entre 35 et 40 % poids/poids de flavanones non-HMG (3-hydroxy-3-méthyl-glutaryl) et
b) au moins 5 % poids/poids de flavanones HMG ; de préférence, ladite composition est constituée de deux ou trois extraits mélangés ou combinés et/ou, de préférence, dans laquelle la teneur en flavanones ne dépasse pas 60 % en poids par rapport au poids total de la composition ; de préférence, dans laquelle ladite composition se présente sous forme micronisée.

**2.** Composition selon la revendication 1, adaptée à un usage pharmaceutique et/ou en tant que complément alimentaire.

**3.** Composition selon la revendication 1 ou la revendication 2, dans laquelle la teneur en flavanones HMG ne dépasse pas 14 % poids/poids, de préférence elle est égale à 5 % ou à 7 %, de préférence dans laquelle ladite composition se présente sous forme micronisée.

**4.** Composition selon l'une quelconque des revendications précédentes, dans laquelle la teneur en flavanones non-HMG est égale à 35 % poids/poids, de préférence dans laquelle la teneur en flavanones HMG est égale à 5 %

poids/poids.

**5.** Composition selon l'une quelconque des revendications précédentes, dans laquelle la teneur en flavanones non-HMG est égale à 35 % poids/poids, la teneur en flavanones HMG est égale à 5 % poids/poids et la composition se présente sous forme micronisée.

**6.** Composition selon l'une quelconque des revendications 1 à 3, dans laquelle la teneur en flavanones est d'au moins 45 % en poids par rapport au poids total de la composition.

**7.** Composition selon la revendication 1, la revendication 2 ou la revendication 6, comprenant :

a) entre 35 et 40 % poids/poids de flavanones non-HMG (3-hydroxy-3-méthyl-glutaryl)
b) au moins 7 % poids/poids de flavanones HMG, de préférence la teneur en flavanones HMG ne dépasse pas 14 % poids/poids, de préférence elle est égale à 7 %.

**8.** Composition selon la revendication 1, la revendication 2, la revendication 6 ou la revendication 7, dans laquelle la teneur en flavanones non-HMG est égale à 38 % poids/poids, de préférence dans laquelle la teneur en flavanones HMG est égale à 7 % poids/poids.

**9.** Composition selon l'une quelconque des revendications 1 à 2, ou 6 à 8, dans laquelle la teneur en flavanones non-HMG est égale à 38 % poids/poids, et la teneur en flavanones HMG est égale à 7 % poids/poids.

**10.** Composition selon l'une des revendications précédentes, dans laquelle l'extrait végétal est choisi parmi le groupe constitué de : l'extrait de bergamote (Citrus Bergamia Risso et Poiteau), l'extrait de pomelo (Citrus Maxima), l'extrait de pamplemousse (Citrus x Paradisi), l'extrait d'orange amère (Citrus aurantium L. var. amara) ou des parties ou fractions de ceux-ci, ou des combinaisons ou mélanges de ceux-ci, de préférence sous forme de jus et/ou d'écorces et/ou de « pastazzo », et/ou dans laquelle ladite composition se compose de :

- un premier extrait comprenant un pourcentage de flavanones compris dans la plage suivante : flavanones non-HMG de 31 à 35 % et flavanones HMG de 5 à 11 % ou de 12 à 15 % en poids par rapport au poids de l'extrait, et
- un deuxième extrait comprenant un pourcentage de flavanones compris dans la fourchette suivante : flavanones non-HMG de 35 à 45 % ou de 45 à 50 % et flavanones HMG de 0 à 5 % en poids par rapport au poids de l'extrait et/ou dans laquelle les flavanones non-HMG sont choisies parmi le groupe constitué de : la naringine, la néoériotricine, la néoéspéridine ou des combinaisons de celles-ci et/ou les flavanones HMG sont choisies parmi le groupe constitué de : la brutieridine, la mélitidine ou des combinaisons de celles-ci, et/ou dans laquelle la composition se présente sous forme solide, semi-solide ou liquide et/ou en association avec des agents anti-dyslipidémiques et/ou hypolipidémiants et/ou avec d'autres ingrédients adaptés à un usage alimentaire ou pharmaceutique, tels qu'un(e) ou plusieurs vitamines, minéraux, enzymes, protéines et/ou d'autres extraits végétaux.

**11.** Composition selon l'une quelconque des revendications précédentes, constituée d'au moins deux extraits présentant chacun un pourcentage connu de flavanones non-HMG et de flavanones HMG, mélangés ou combinés de manière à obtenir :

- une composition **caractérisée par** une teneur en flavanones non-HMG de 35 % poids/poids et une teneur en flavanones HMG égale à 5 % poids/poids, dans laquelle ladite composition se présente sous forme micronisée, ou
- une composition **caractérisée par** une teneur en flavanones non-HMG de 38 % poids/poids et une teneur en flavanones HMG égale à 7 % poids/poids.

**12.** Composition pharmaceutique comprenant la composition selon l'une quelconque des revendications 1 à 11 et au moins un excipient et/ou un véhicule acceptable sur le plan pharmaceutique.

**13.** Complément alimentaire comprenant la composition selon l'une quelconque des revendications 1 à 11.

**14.** Composition selon l'une quelconque des revendications 1 à 11, ou la composition pharmaceutique selon la revendication 12, ou le complément alimentaire selon la revendication 13, destinés à un usage médical, de préférence destinés à la prévention et/ou au traitement des maladies cardiométaboliques, des dyslipidémies, de la résistance à

l'insuline, du diabète de type 2, de la NAFLD (stéatose hépatique non alcoolique)/NASH (stéatohépatite non alcoolique), de l'hypertension, de l'obésité, de la graisse viscérale, afin de lutter contre les troubles/facteurs de risque typiques de l'obésité et des maladies cardiovasculaires, tels que la glycémie, les triglycérides et le cholestérol.

**15.** Utilisation non thérapeutique de la composition selon l'une des revendications 1 à 11 ou du complément alimentaire selon la revendication 13 en tant qu'ingrédient de base dans des préparations de compléments alimentaires ou de médicaments.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **DI DONNA et al.** *J Nat Prod.*, 2009
- **LEOPOLDINI et al.** *J. Agric. Food Chem.*, 2010
- **JANDA et al.** *PharmaNutrition*, 2016
- **PU et al.** *Arch Biochem Biophys.*, 2012
- **CHAUMEIL J. C.** Micronization: a method of improving the bioavailability of poorly soluble drugs.. *Methods and findings in experimental and clinical pharmacology*, 1998, vol. 20 (3), 211-215
- Remington: The Science and Practice of Pharmacy. Lippincott Williams & Wilkins, 2000
- **BALLISTRERI et al.** Evaluation of lipid and cholesterol-lowering effect of bioflavonoids from bergamot extract. *Natural Product Research*, 2020